(19)

Europäisches
Patentamt
European
Patent Office
Office européen
des brevets

(11) **EP 2 324 762 A1**

(12) **EUROPEAN PATENT APPLICATION**

(43) Date of publication:
   **25.05.2011 Bulletin 2011/21**

(51) Int Cl.:
   **A61B 5/0205** (2006.01)    **A61B 5/22** (2006.01)

(21) Application number: **09176608.9**

(22) Date of filing: **20.11.2009**

(84) Designated Contracting States:
   **AT BE BG CH CY CZ DE DK EE ES FI FR GB GR HR HU IE IS IT LI LT LU LV MC MK MT NL NO PL PT RO SE SI SK SM TR**
   Designated Extension States:
   **AL BA RS**

(71) Applicant: **Koninklijke Philips Electronics N.V.**
   **5621 BA Eindhoven (NL)**

(72) Inventors:
   • **Maeueler, Sebastian**
    **5600 AE, EINDHOVEN (NL)**

   • **Ter Horst, Herman, J.**
    **5600 AE, EINDHOVEN (NL)**
   • **Tokmakoff, Andrew, A.**
    **5600 AE, EINDHOVEN (NL)**
   • **Burza, Marek, K.**
    **5600 AE, EINDHOVEN (NL)**

(74) Representative: **Damen, Daniel Martijn**
   **Philips Intellectual Property & Standards**
   **P.O. Box 220**
   **5600 AE Eindhoven (NL)**

(54) **Fitness test system**

(57)    The present invention relates to a fitness test system for determining a fitness test result indicative of a user's fitness, for a plurality of components of physical fitness such as endurance, strength, flexibility, balance, and body composition. The fitness test system comprises a computing module (1) for accessing a collection of fitness tests and one or more peripheral devices (20-23), the one or more peripheral devices being communicatively connected to the computing module, and the one or more peripheral devices being adapted to measure one or more input parameters. The computing module is programmed for upon selection of a fitness test to determine the group of input parameters associated with the fitness tests and based on the determined input parameters to calculate and/or present a fitness test result indicative of a user's fitness related to the associated com- ponent of physical fitness.

FIG. 2

EP 2 324 762 A1

**Description**

FIELD OF THE INVENTION

The present invention relates to a fitness test system for determining a fitness test result that is indicative of a user's physical fitness.

BACKGROUND OF THE INVENTION

Physical fitness is a crucial factor for a healthy life; and people who are physically inactive risk different kinds of diseases. While most people are aware of the importance of being physically fit, the number of people suffering from health problems related to physical inactivity is continuously increasing.

A number of factors are important in connection with physical fitness and physical activity, one factor being motivation and another factor being knowledge about a person's level of fitness. The ability to monitor and test the personal level of fitness is believed to be an important motivating factor. However, in order to understand and use test results to improve or maintain a given fitness level, it is important that the fitness test properly reflects the fitness state of the person taking the test.

There is widespread agreement among experts that there are five crucial components of physical fitness as far as it relates to health: endurance, strength, flexibility, balance, and body composition. Many different fitness tests are available, however these tests are hard to do without assistance and involve complex data collection and complex calculations to arrive at the person's fitness level. To overcome at least some of these drawbacks, fitness centers, for example, offer professional help e.g. in the form of personal help or specialized test equipment. However, this is often expensive and inflexible for the consumer.

The US patent application no. 2007/0232454 AI discloses a fitness assessment system suitable for personal use. The system includes a server system set up to receive data from a user, and based on the data generates a fitness score in a plurality of health-related fitness categories.

SUMMARY OF THE INVENTION

The inventors of the present invention have realized that a comprehensive test of a person's overall fitness level cannot be done by taking only a single test; and it would be advantageous to achieve a system for comprehensively and objectively measuring a plurality of components of a person's fitness level. It would be desirable to be able to use such a system in a home-setting or in another setting where expert guidance is unavailable, while still maintaining a high level of confidence in the obtained results. It would also be desirable to provide a system which can be realized with a limited number of tools, and which avoids tedious and potentially erroneous data registration and collection by the user. In general, the invention preferably seeks to mitigate, alleviate or eliminate one or more disadvantages of the prior art singly or in any combination.

To better address one or more of these concerns, in a first aspect of the invention a fitness test system is presented that comprises:

a computing module for accessing a collection of fitness tests, the collection of fitness tests comprises fitness tests related to a plurality of components of physical fitness, each fitness test is associated with a group of input parameters to be determined, and wherein each input parameter is associated with one or more peripheral devices and/or with the computing module;

one or more peripheral devices, the one or more peripheral devices being communicatively connected to the computing module, and the one or more peripheral devices being adapted to measure one or more input parameters; wherein the computing module is programmed for upon selection of a fitness test to determine the group of input parameters associated with the fitness test and based on the determined input parameters to calculate and/or present a fitness test result indicative of a user's fitness related to a component of physical fitness.

By providing a fitness system which integrates the fitness tests to be performed, the input parameters to be determined, and the device(s) that are used to perform the tests in order to determine the input parameters and the calculation of the fitness result, a system is provided which is suitable for use by persons without expert knowledge on physical fitness and which therefore can be used without guidance from experts to obtain results with a high level of confidence. Users can thereby assess and monitor their physical fitness privately without the need for professional support. By proper selection of the individual tests, it can be ensured that the tests are selected based on scientific evidence instead of merely on personal preferences. Moreover, by communicatively arranging the peripheral devices and the computing module it may be assured that the system possesses a high flexibility in the specific devices that may be used. The fitness test system may further comprise display facilities or other facilities, e.g. implemented by one of the peripheral devices, on which the calculated test results can be presented. Preferably, the calculated fitness results are presented to the user on a display giving the user visual feedback. The fitness tests may be associated with a health-related component of physical fitness. An example of a component of physical fitness that is not health-related, but performance-related, is speed. A component of physical fitness may also be referred to as an aspect of or a dimension of physical fitness.

In general, the fitness test system may be suitable for use in a home-setting, however the system may also be used in connection with an intended use, e.g. in connection with medical intended use. For example in connection with fitness assessment performed or evaluated in connection with a patient's general health state done by a general practitioner or other medical practitioner. Moreover, the system may advantageously be used by schools or other institutions where a fitness state may be evaluated or followed in connection with regular assessments. In general, the system may be used in connection with any type of purpose where a fitness test with a high level of confidence in the obtained results is desirable.

Generally, the collection of fitness tests comprises fitness tests related to a plurality of components of physical fitness. By testing a plurality of different components of physical fitness a multi-dimensional test result may be obtained which assists the user in identifying components which are at satisfactory level as well as components which need improvement. The user is thereby provided with an outcome which can assist the user in deciding upon a direction for further workout. In this respect, a plurality of components is to be understood as two or more components. The system can for example also advise the user to improve his fitness with respect to a component of fitness where the test result is very low compared with the average result for the age and gender group of the user. Advantageously, the collection of fitness tests comprises tests related to the following components of physical fitness: cardiovascular endurance, muscle strength, flexibility, balance, body composition. These five components of physical fitness are the key components with respect to the person's health state. By testing all five components, a comprehensive test which reflects the level of physical fitness of the user can be provided.

Advantageously, the computing module is programmed for automatic coordination of and interaction with peripheral devices to determine the input parameters and calculate the fitness test result. Non-automated measurement and registration of test results is a very error prone and tedious task for the user. By ensuring that data is collected automatically and that the collected data is automatically used by the computing module, it can be ensured that data is registered correctly and that the result is calculated correctly. Moreover, the user is not de-motivated by the task of registering measurement results.

In an advantageous embodiment, at least one of the peripheral devices is suitable for use in connection with tests related to two or more components of physical fitness.

In an advantageous embodiment, at least one of the peripheral devices is an exercise mat which contains one or more sensors. An exercise mat is versatile in its use, thereby rendering it suitable to be used in connection with a large number of fitness tests and thus makes it possible to limit the number of peripheral devices.

In an advantageous embodiment, at least one of the peripheral devices is a distance detecting device suitable for measuring the distance between different body parts e.g. between the user's fingers and/or between the user's fingers and toes. However, other distances such as waist circumference, height, etc. could be measured with the distance detecting device. In an important embodiment, the fitness system comprises an exercise mat and a distance detecting device, and optionally one or more additional peripheral devices. Both an exercise mat and a distance detecting device can be made by use of a low bill of materials. However, the invention is not limited to use of specific types of peripheral devices.

Advantageously, one or more of the peripheral devices or the computing module comprise or is connected to a device capable of handling inputs or providing feedback selected from the group of: audio, visual, audiovisual, haptic, tactile, voice recognition, touch screen, and tangible, or any combination thereof. By handling input or providing feedback, the system may be rendered more user-friendly, as well as more interesting to use.

Advantageously, the computing module may further be adapted for accessing a user's data associated with a user of the fitness test system, and further basing the calculation of the fitness test result on the user's data. By basing the calculation of the test result on user data, an even more detailed and personal fitness evaluation can be made.

In general, the computing module may be implemented into at least one of the peripheral devices. However in important embodiments, the computing module is implemented into a handheld computing device or into the exercise mat.

Advantageously, the fitness test is selected by selecting a component of physical fitness. By selecting a component of physical fitness, for example muscle strength, a representative set of fitness tests, for example the push-up test, the curl-up test and the wall-sit test, is selected and preferably used for execution by the user.

In a second aspect of the invention a computer program product is presented that, when running on a computer, is adapted to:

- accessing a collection of fitness tests, the collection of fitness tests comprises fitness tests related to a plurality of components of physical fitness, each fitness test is associated with a group of input parameters to be determined, and wherein each input parameter is associated with one or more peripheral devices and/or with the computer program product;

- coordinate control of and interaction with the one or more peripheral devices, the one or more peripheral devices being communicatively connected to the computer program product, and the one or more peripheral devices being adapted to measure one or more input parameters;

wherein the computer program product is programmed for upon selection of a fitness test to determine the group of input parameters associated with the fitness test and based on the determined input parameters to calculate and/or present a fitness test result indicative of a user's fitness related to a component of physical fitness.

The computer program product may implement the functionality of the fitness test system in accordance with the first aspect of the invention. The computer program product may be a unified product implemented into a single computing module or separated computer program product implemented into a distributed computing module. In an important embodiment the computer program product is implemented into such a device as a handheld computing device, e.g. in the form of a mobile or cellular phone, such as a so-called smart phone, or a portable media player such as an MP3 player, to render the handheld device with the functionality of the computing module of the first aspect of the present invention. It is an advantage of the present invention that it may be implemented by devices or platforms with low computing power.

In general the various aspects of the invention may be combined and coupled in any way possible within the scope of the invention. These and other aspects, features and/or advantages of the invention will be apparent from and elucidated with reference to the embodiments described hereinafter.

BRIEF DESCRIPTION OF THE DRAWINGS

Embodiments of the invention will be described, by way of example only, with reference to the drawings, in which

Fig. 1 illustrates a schematic embodiment of functional elements of the fitness test system;
Fig. 2 illustrates an exemplary embodiment of a fitness test system making use of a handheld computing device, an exercise mat and a distance detecting device;
Fig. 3 schematically illustrates a peripheral device in the form of an exercise mat;
Fig. 4 schematically illustrates a peripheral device in the form of a distance detecting device; and
Fig. 5 illustrates an example of a graphical visualization of the fitness test result.

DESCRIPTION OF EMBODIMENTS

The present invention is related to a fitness test system suitable for use without assistance from an expert in physical fitness testing. It is the intention of embodiments of the present invention to provide an easy-to-use system which in embodiments is capable of providing guidance to the user, perform the actual measurements needed in order to perform the test, as well as performing the final evaluation, that is to calculate fitness results. In a general embodiment, the overall functionality of the fitness system is that the user selects a test or is prompted by the system which test should be next; the user receives instructions on how to perform the test, e.g. via visuals and sound and executes the test with the help of associated peripheral devices; the execution of the test is monitored by the system through the use of sensors; upon execution of the test, the user is presented with the test result. In embodiments, the user typically selects a complete test, for all components of physical fitness that can be handled by the system, and the computing module handles the execution of the associated fitness tests. However, the user may also select one or more components of physical fitness and the computing module handles the selection of suitable, representative fitness tests associated to each selected component. Moreover, the user may select an individual fitness test.

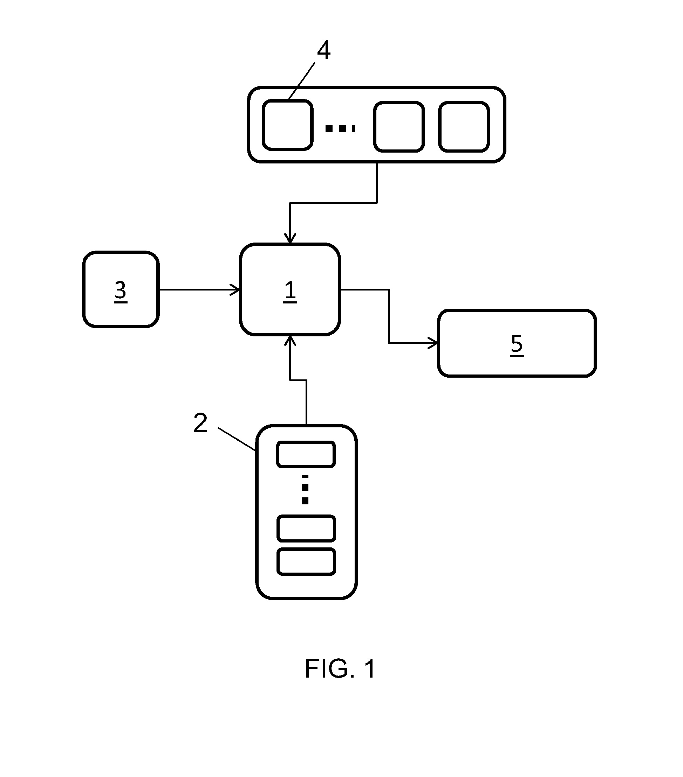

Figure 1 illustrates a schematic embodiment of functional elements of the fitness test system. The system comprises a computing module 1. The computing module is connected to a collection of fitness test 2, where the collection contains tests related to a plurality of components of physical fitness. The fitness test may be associated with a health-related component of physical fitness, and each fitness test is associated with a group of input parameters to be determined in order to calculate a test result. The computing module is moreover capable of accessing user data 3, such as age, gender, weight, height, etc., e.g. from a storage unit to which the computing module is connected or from interaction with the user. The system further comprises one or more peripheral devices 4, however typically two, three or more peripheral devices are used. The peripheral devices are adapted to measure one or more input parameters. Moreover, one or more of the input parameters may be determined by the computing module 1, for example, time keeping may suitably be handled by the computing module. Moreover, the fitness test system may comprise or be connected to a storage unit for storing test results, so that the user can monitor the progress over time or see a trend.

The computing module 1 is programmed for upon selection of a fitness test, either by the user or as part of a test program, to determine the group of input parameters associated with the fitness test 2 and based on the input parameters determined by the computing module itself or received through the communication line, and optionally the user data, to calculate a fitness result 5 indicative of a user's fitness related to the associated health-related component.

Embodiments of the fitness system and associated tests are now described in terms of specific examples of how a test may be performed.

In an embodiment, the collection of fitness tests comprises tests related to the following components of physical fitness: cardiovascular endurance, muscle strength, flexibility, balance, body composition. In order to be able to perform a complete fitness test, the computing module is programmed for execution of at least one fitness test associated with each of these components of physical fitness. Each component of physical fitness may even have more associated tests. Also alternative tests may be associated with the components of physical fitness.

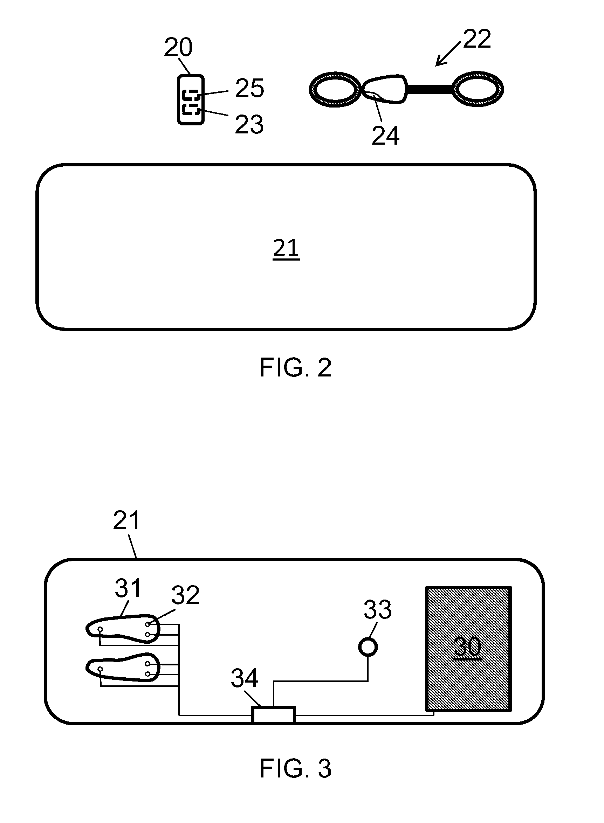

Figure 2 illustrates an exemplary embodiment of a fitness test system making use of three peripheral devices: a handheld computing device 20, an exercise mat 21 and a distance detecting device 22. In this embodiment, a computing program product 25 in the form of a computer application is implemented in the handheld computing device 20. However in other embodiments the computing functionality may be implemented in other peripheral devices, such as in the exercise mat 21. In the illustrated embodiment, the handheld computing device further comprises a GPS unit 23 and the distance measuring device comprises an oximeter 24. The handheld computing device may e.g. be a smart phone comprising the computing facilities to run a computer program stored thereon. In other embodiments the computing module may be distributed over a number of the peripheral devices, e.g. to form a peer-to-peer network with distributed control. Additionally, the computing module may be communicatively connected to a web service or web application, or it may include a web service or web application, e.g. to store results and possibly to participate in calculation of partial results or even end results. It is also possible that a client/server architecture is used wherein the presentation of the results is remote to the calculation of the results.

To initiate a fitness test, the user runs a computer program implemented by the handheld computing device. In an embodiment, the computing module stores instructions associated with the tests and presents the instructions to the user to orchestrate the tests. These instructions may comprise instructions on how to do warm up exercise(s), which tests are to be performed as well as how to perform the tests.

In this example, the tests are performed in the following order: cardiovascular endurance, muscle strength, flexibility, balance and body composition. The invention is however not limited to this order, and different orders may be used in different implementations. In a particular embodiment, tests which require that the user is not too tired may be performed early in the succession of tests, in an example the balance test may be performed first, then the muscle strength tests, then flexibility tests and next the endurance test. The body composition test may be performed before or after these tests depending on the specific test: a BIA test should be done before the other fitness tests and should, when repeated, be done at the same time of the day.

Cardiovascular endurance

There exist different alternatives for testing the endurance. The endurance is tested by estimating the maximal oxygen consumption ($VO_2max$) of the user. This can be tested by a run test, a walk test or a step test. In an embodiment, the computing module presents an enquiry to the user regarding which test to perform. In an example the user may select the run test. The run test may be the standard Cooper test where a person runs as fast as possible for 12 minutes. It is known that a good approximation of the $VO_2max$ value can be obtained by using the following formula:

$$VO_2max = (D- 504.9)/44.73$$

with D being the run distance in meters.

Thus upon selection of the endurance fitness test related to run, the computing module accesses the collection of fitness tests to determine that in order to perform the test, the following input parameters are needed: time and distance and which peripheral devices are to be used for determining these parameters. Moreover, user data related to age and gender may be needed in order to evaluate the test result. The test may be performed by instructing the user to find an area suitable for running 12 minutes. When this is done, the computing module instructs the user to start running. Any type of feedback, e.g. audiovisual may be provided during the run. The time keeping may be done by the computing module, and once 12 minutes have passed, the computing module accesses the GPS unit to obtain the covered distance. This distance is then used to calculate the $VO_2max$ value, and the computing module consults a look-up table which rates $VO_2max$ values in relation to gender and age. For example, the look-up table may comprise listings of average values for persons of a given age, as well as a gradation that may be used for rating of the performance. Thus for the run test, the fitness index is calculated by first calculating the $VO_2max$ from the measured distance, and then deducing from a look-up table listing, for example on a scale from 1 to 5, how well the measured $VO_2max$ is for a person of a given age and gender.

Another example of the endurance test is the walk test. In the walk test, the user walks 2 km and the walk time is detected. The $VO_2max$ can be approximated using the formula

$$VO_2max = 116.2 - 2.98 * T - 0.11 * fh - 0.14 * A - 0.39 * BMI$$

for women. A similar formula exists for male users.

In order to determine the $VO_2max$ from the walk test the following input parameters are needed: the time, $T$, the final heart rate, $fh$, the age, $A$, and the BMI (which can be calculated from the user data). Thus in this embodiment, a peripheral device capable of measuring the heart rate is used. In an embodiment, the distance measuring device 22 comprises an oximeter 24 to be used on the user's finger.

Again based on a look-up table, the determined $VO_2max$ can be rated.

An even further alternative of the endurance test is the step test, in the form of the Canadian aerobic fitness test.

Muscle strength

The muscle strength may in embodiments be tested by three muscle tests relating to the upper body, lower body and core body, respectively. The fitness result related to these three tests can be obtained by averaging the result of each of the three tests. In an embodiment is the muscle strength tests performed by using a peripheral device in the form of an exercise mat 21, communicatively connected to the handheld device 20. The exercise mat may comprise pressure sensors and proximity sensors located in the mat. Additionally, the exercise mat may comprise a display, which may be used as an alternative to the display on the handheld computing module in order to present instructions and information to the user. The exercise mat is further disclosed in connection with Figure 3.

The strength of the upper body can be tested by counting the number of push-ups that can be performed according to a prescribed rhythm. In order to perform the test, the user may position his or her hands on the exercise mat. By use of audio or visual feedback, the user can be instructed as to the rhythm of the push-ups. Moreover, by use an ultrasonic proximity sensor, the number of push-ups can be counted. The user performs the number of push-ups that he or she is able to do, and based on the gender and age, the obtained number is used to evaluate the result. As an example of a table that may be used for evaluation of the test result, the following table relates to the number of push-ups for men:

| Ratings | Nr. of push-ups performed (men) | | | | |
|---|---|---|---|---|---|
| Age (years) | 20-29 | 30-39 | 40-49 | 50-59 | 60+ |
| well above average | >=36 | >=30 | >=25 | >=21 | >=18 |
| above average | 29-35 | 22-29 | 17-24 | 13-20 | 11-17 |
| average | 22-28 | 17-21 | 13-16 | 10-12 | 8-10 |
| below average | 17-21 | 12-16 | 10-12 | 7-9 | 5-7 |
| well below average | <=16 | <=11 | <=9 | <=6 | <=4 |

In a similar manner the core body strength is tested, however for the core body strength it is the number of curl-ups which is counted. Pressure sensor(s) or a proximity sensor in the exercise mat may be used to count the number.

The lower body strength may be tested by performing the wall-sit test. In the wall-sit test, the user puts both feet on the exercise mat and lean up against a wall to put the body in a sitting position. One of the feet is lifted from the exercise mat, and the time during which this position can be maintained is measured. After a rest period, the other leg is tested. The pressure sensor is used to detect when a foot is lifted and put down again, and either the computing module, or a module of the exercise mat, determines the time.

Flexibility

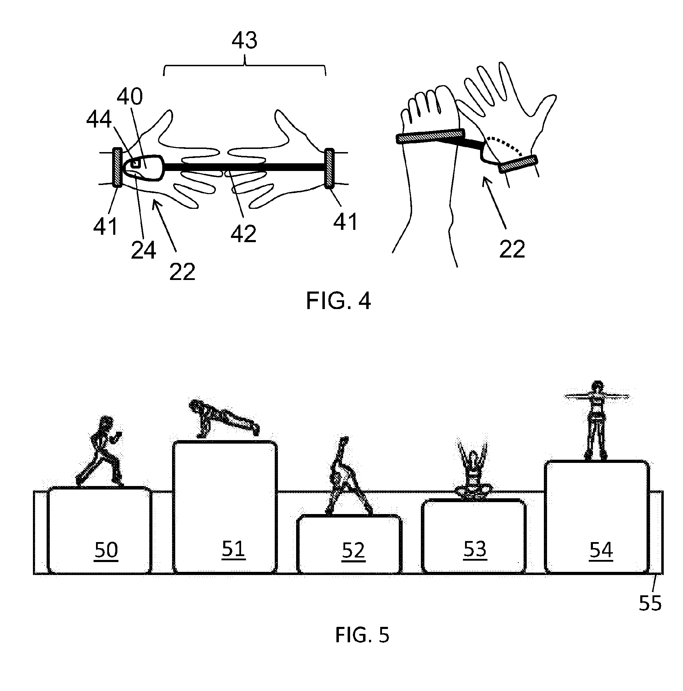

The flexibility test may in embodiments be tested by averaging the test result of two flexibility tests: a sit-and-reach test and a shoulder flexibility test. In an embodiment, the flexibility tests are performed by using a peripheral device in the form of an automated distance measuring device. The measuring device is further disclosed in connection with Figure 4.

The sit-and-reach test is used to measure lower body flexibility. The test involves sitting on the floor with legs out straight ahead and toes pointing up. The measuring device is attached to and spanned between a wrist and a foot. The user reaches forward as far as possible and then the distance between the toes and the finger tips is determined.

The shoulder flexibility test is used to measure upper body flexibility. It is done by putting both hands behind one's back, one over the shoulder and one under the shoulder and reaching towards each other. The measuring device is attached to and spanned between the two wrists and the input to the test is the minimum distance between the finger tips reached during the test.

Balance

The balance test may in embodiments be tested by a single test, the Stork balance stand test. In an embodiment, the balance test is performed by using a peripheral device in the form of the exercise mat. The test is used to measure balance ability of the body. It is done by standing on the ball of one foot while the hands are kept on the hips and the foot of the non-supporting leg touches the knee of the supporting leg. The input is the time during which this position is maintained statically or without much movement. This can be determined by use of pressure sensors in the exercise mat.

Body composition

The body composition test is to determine body composition, which is characterized for example by fat mass, muscle mass and water content. This can be done by using standard equipment for bioelectrical impedance analysis (BIA) combined with anthropometric data such as weight. A BIA-device may be included in or as one of the peripheral devices. In an embodiment, partial information on body composition may be obtained by use of the user's body-mass index (BMI) and the waist circumference; determination of BMI and waist circumference cannot be viewed as a sufficient test on body composition. BMI data can be determined based on user data in the form of weight and height.

Having described the various tests, examples of peripheral devices are further disclosed in the following. The above described tests constitute a complete set of tests in order to test the physical fitness of all five health related components. Other or alternative tests may be envisioned. The tests may be accessed by the computing module e.g. by accessing a storage unit to which it is connected. The collection of tests may also be updated or changed, e.g. by storing an updated collection on the storage unit.

Figure 3 schematically illustrates a peripheral device in the form of an exercise mat 21. In an embodiment, the exercise mat comprises a display 30, such as a flexible display, and a number of sensors in the mat. In the illustrated embodiment, two types of sensors are present, pressure sensors and a proximity sensor. The pressure sensors 32 may be located in marked areas 31 intended for positioning of the feet. In the illustrated embodiment, three sensors per foot are used, however other numbers and other configuration of sensors may be used. Additional pressure sensors may also be placed at other positions within the mat. In general even a net or grid of sensors may be positioned in an area of or in the entire exercise mat. Moreover, a proximity sensor 33 is illustrated. The proximity sensor may be an ultrasonic sensor capable of detecting distance, and thus of variations in distance, for detecting and counting the number of push-ups and possibly curl-ups. In an embodiment, the curl-ups may also be counted by use of pressure sensors, e.g. the sensors 32 in the foot area or dedicated sensors placed at appropriate positions. As an alternative to pressure sensors, load cells may be used. The sensors and the display may be connected to a controller 34 positioned in the mat. The controller may also be implemented by the computing module, in which case, the sensors and the display are directly connected to the computing device or connected to a communication unit. In an embodiment, the computing module may also be, or additionally be, provided in the mat. The mat may be wirelessly connected to other peripheral devices; alternatively the mat may be connected via a cable connection.

Figure 4 schematically illustrates a peripheral device in the form of a distance detecting device. The figure schematically illustrates the attachment of the distance measuring device between the user's wrists and between the user's wrist and foot. In an embodiment, the device comprises a central unit 40 and two bands 41 suitable for attachment to a hand or foot. One band is attached to the central unit 40 and the other band is attached to a distance detecting unit 42, such as a cable connected also to the central unit 40. The device may implement a calibration routine to be able to use the device with different people. For the shoulder flexibility test this may be done by touching the tips of the middle fingers while keeping the forearms including hands in one straight line and by detecting the length 43 of the cable extension. Additionally the device may comprise an oximeter 24 for measuring the heart rate of the user from the user's finger. Similar to the mat, the distance detecting unit may support wireless communication or cable connection to the computing module via a communication unit 44.

A number of other peripheral devices may also be envisioned. These devices may be implemented as stand-alone devices or built together with other devices to increase the functionality with fewest possible device units. As an example, a video recorder with associated image analysis software may be used to detect distances or counting the number of repetitions. Distance may also be measured by means of an ultrasound-based distance measuring device. Pulse rate may be measured by means of belt or strap attached to the chest. Run or walk distance may be measured by use of an accelerometer or other motion detection devices. An accelerometer may also be used to count the number of repetitions when attached to the chest. Moreover, pressure sensitive cover sheets for steps on a stair may be provided to perform the step test. The system may provide connectivity to an external display, such as a computer screen or a TV, to provide large high-resolution display facilities. Moreover a modem TV possesses sufficient computing power to implement the computing module. Additionally a scale may also be used as a peripheral device. The scale may be used to detect the weight of the user and also be used for the balance test, e.g. the Stork balance stand test. A scale may be provided which include one or more sensor(s). A scale may be augmented with additional functionality needed to perform many of the fitness tests.

Moreover, from analysis of the received sensor signals, the system may further be programmed to check the correctness of the performed movement, as well as to give instructions on how to perform a given test in the event it is detected that the user's movements are incorrect.

The result of a fitness test may be represented by a fitness index in the form of a numerical score value, such as a value ranging from 0 to 5 for each of the components of physical fitness, as well as a combined (average or weighted average) aggregate fitness index. The result may be presented graphically, an example of which is provided in Figure 5. Working with fitness results in this way facilitates comparison between the health-related components, as well as over time. Moreover, the fitness results may be compared to average test results for the user's age and gender group.

Figure 5 illustrates an example of a graphical visualization of the fitness test result. Each health-related component is presented by a column: cardiovascular endurance 50, muscle strength 51, flexibility 52, balance 53, body composition 54 and an aggregate fitness index 55.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Fitness test system comprising:

    - a computing module (1) for accessing a collection of fitness tests (2), the collection of fitness tests comprises fitness tests related to a plurality of components of physical fitness, each fitness test is associated with a group of input parameters to be determined, and wherein each input parameter is associated with one or more peripheral devices (4) and/or with the computing module;
    - one or more peripheral devices (4), the one or more peripheral devices being communicatively connected to the computing module (1), and the one or more peripheral devices being adapted to measure one or more input parameters;
    wherein the computing module (1) is programmed for upon selection of a fitness test to determine the group of input parameters associated with the fitness test and based on the determined input parameters to calculate and/or present a fitness test result (5) indicative of a user's fitness related to a component of physical fitness.

2. The fitness test system according to claim 1, wherein the computing module (1) is further programmed for executing a fitness test by accessing instructions associated with the fitness test and presenting the instructions to the user.

3. The fitness test system according to claim 1, wherein the collection of fitness tests (2) comprises tests related to the following components of physical fitness:

    cardiovascular endurance, muscle strength, flexibility, balance, body composition, and
    wherein the computing module is programmed for determining input parameters related to at least one fitness test associated with each of the following components of physical fitness: cardiovascular endurance, muscle strength, flexibility, balance, body composition and an aggregate fitness result.

4. The fitness test system according to claim 1, wherein the computing module (1) is programmed for automatic coordination of and interaction with peripheral devices (4) to determine the input parameters and calculate the fitness test result.

5. The fitness test system according to claim 1, wherein the computing module (1) is implemented into at least one of the peripheral devices (4).

6. The fitness test system according to claim 1, wherein at least one of the peripheral devices is an exercise mat (21), wherein the exercise mat comprises one or more sensors (32, 33), the one or more sensors being communicatively connected to a controller (34) located in or on the mat or implemented by the computing module (1).

7. The fitness test system according to claim 6, wherein the exercise mat further comprises a display (30).

8. The fitness test system according to claim 6, wherein the one or more sensors comprise pressure sensors (32) contained by the exercise mat (21).

9. The fitness test system according to claim 6, wherein the one or more sensors comprise a proximity sensor (33).

10. The fitness test system according to claim 1, wherein at least one of the peripheral devices is a distance detecting device (22).

11. The fitness test system according to claim 1, wherein one or more of the peripheral devices or the computing module comprises or is connected to a device capable of handling inputs or providing feedback selected from the group of: audio, visual, audiovisual, haptic, tactile, voice recognition, touch screen, and tangible, or any combination thereof.

12. The fitness test system according to claim 1, wherein the computing module is implemented into a handheld computing device (20).

13. The fitness test system according to claim 1, wherein the computing module is further adapted for accessing a user's data (3) associated with a user of the fitness test system, and for further basing the calculation of the fitness test result on the user's data.

14. The fitness test system according to claim 1, wherein the fitness test is selected by selecting a component of physical fitness.

15. A computer program product (25), when running on a computing device, being adapted to:

   - accessing a collection of fitness tests (2), the collection of fitness tests comprises fitness tests related to a plurality of components of physical fitness, each fitness test is associated with a group of input parameters to be determined, and wherein each input parameter is associated with one or more peripheral devices (4) and/or with the computer program product (25);
   - coordinate control of and interaction with the one or more peripheral devices (4), the one or more peripheral devices being communicatively connected to the computer program product, and the one or more peripheral devices being adapted to measure one or more input parameters;
   wherein the computer program product is programmed for upon selection of a fitness test to determine the group of input parameters associated with the fitness test and based on the determined input parameters to calculate a fitness test result indicative of a user's fitness related to a component of physical fitness.

4

3 1 5

2

FIG. 1

20

25

23

22

24

21

FIG. 2

21

31 32

33

34

30

FIG. 3

FIG. 4

FIG. 5

**EP 2 324 762 A1**

Europäisches
Patentamt

European
Patent Office

Office européen
des brevets

# EUROPEAN SEARCH REPORT

Application Number

EP 09 17 6608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 110 073 A (SAUR JOHN [US] ET AL) 29 August 2000 (2000-08-29) * abstract; figures 1,16 * * column 2, lines 13-67 * * column 4, line 30 - column 5, line 4 * * column 5, line 65 - column 7, line 6 * ----- | 1,2,4-8, 11-15 | INV. A61B5/0205 A61B5/22 |
| X | GB 2 432 282 A (HWANG YUH-SWU [TW]) 16 May 2007 (2007-05-16) * abstract; figures 2,4,10-13,15 * * page 4, line 2 - page 5, line 12 * * page 8, line 15 - page 11, line 10 * * page 13, lines 18-26 * * page 15, line 13 - page 16, line 5 * * page 21, line 23 - page 22, line 1 * * page 20, line 11 - page 39, line 14 * ----- | 1,2,4, 10-13 | |
| X | WO 2006/111687 A1 (UNIV VICTOR SEGALEN BORDEAUX 2 [FR]; OWNWAY [FR]; LOFI ALAIN [FR]; TRO) 26 October 2006 (2006-10-26) * abstract; figures 1,5 * * page 1, line 19 - page 2, line 2 * * page 2, lines 14-27 * * page 3, line 14 - page 4, line 3 * * page 4, lines 4-21 * * page 4, line 22 - page 5, line 14 * * page 5, line 15 - page 7, line 11 * * page 7, line 12 - page 8, line 7 * * page 8, line 8 - page 9, line 8 * * page 11, line 12 - page 12, line 5 * ----- -/-- | 1,3,4, 6-8,10, 11,13 | |

TECHNICAL FIELDS SEARCHED (IPC)

A61B

The present search report has been drawn up for all claims

| Place of search | Date of completion of the search | Examiner |
|---|---|---|
| Munich | 29 March 2010 | Medeiros Gaspar, Ana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt
European
Patent Office
Office européen
des brevets

**EUROPEAN SEARCH REPORT**

Application Number

EP 09 17 6608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 435 315 A (MCPHEE RON J [US] ET AL) 25 July 1995 (1995-07-25)<br><br>* abstract; figures 1,4 *<br>* column 1, line 34 - page 2, line 33 *<br>* column 2, line 56 - column 3, line 4 *<br>* column 3, lines 22-30 *<br>* column 4, lines 30-36 *<br>* column 5, lines 25-27 *<br>* column 5, lines 51-57 *<br>* column 6, lines 30-44 *<br>----- | 1,3,4, 6-8,11, 13,14 | |
| X | WO 2006/053000 A2 (SPARQ INC [US]; CHAPA RODOLFO JR [US]; ARJOMAND HAMID G [US]; BRAUN AD) 18 May 2006 (2006-05-18)<br>* abstract; figures 13c,14-19 *<br>* page 3, line 13 - page 4, line 3 *<br>* page 7, lines 5-9,25-30 *<br>* page 9, lines 9-28 *<br>* page 10, lines 8-16 *<br>* page 13, lines 19-23 *<br>* page 14, lines 12-18 *<br>* page 17, lines 15-31 *<br>----- | 1,4, 10-13 | |
| A | GB 2 185 109 A (FROST BARRY JOHN) 8 July 1987 (1987-07-08)<br>* abstract; claims 1,2; figure 8 *<br>* page 1, lines 5-21,54-56,118-121 *<br>* page 2, lines 22-25 *<br>----- | 1,15 | TECHNICAL FIELDS SEARCHED (IPC) |
| A | US 6 010 452 A (HARCOURT KRISTIAN L [AU]) 4 January 2000 (2000-01-04)<br>* abstract; figure 1 *<br>----- | 1,15 | |

The present search report has been drawn up for all claims

| Place of search | Date of completion of the search | Examiner |
|---|---|---|
| Munich | 29 March 2010 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 17 6608

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2010

| Patent document cited in search report | | Publication date | Patent family member(s) | | Publication date |
|---|---|---|---|---|---|
| US 6110073 | A | 29-08-2000 | NONE | | |
| GB 2432282 | A | 16-05-2007 | NONE | | |
| WO 2006111687 | A1 | 26-10-2006 | FR 2884702 A1 | | 27-10-2006 |
| US 5435315 | A | 25-07-1995 | NONE | | |
| WO 2006053000 | A2 | 18-05-2006 | EP 1830931 A2 | | 12-09-2007 |
| | | | US 2007272011 A1 | | 29-11-2007 |
| GB 2185109 | A | 08-07-1987 | NONE | | |
| US 6010452 | A | 04-01-2000 | NONE | | |

EPO FORM P0459

For more details about this annex : see Official Journal of the European Patent Office, No. 12/82

**EP 2 324 762 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070232454 A1